(19) Europäisches Patentamt
European Patent Office
Office européen des brevets

(11) **EP 4 020 496 A1**

(12) **EUROPEAN PATENT APPLICATION**

(43) Date of publication:
**29.06.2022 Bulletin 2022/26**

(51) International Patent Classification (IPC):
***G16H 50/70*** *(2018.01)*

(21) Application number: **21196267.5**

(52) Cooperative Patent Classification (CPC):
**G16H 50/70**

(22) Date of filing: **13.09.2021**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **24.12.2020 TW 109145888**

(71) Applicants:
• **Acer Incorporated
New Taipei City 221 (TW)**
• **National Yang Ming Chiao Tung University
Hsinchu 30010 (TW)**

(72) Inventors:
• **CHEN, Pei-Jung
221 New Taipei City (TW)**
• **TSAI, Tsung-Hsien
221 New Taipei City (TW)**
• **CHEN, Liang-Kung
30010 Hsinchu (TW)**
• **HUANG, Shih-Tsung
10617 Taipei (TW)**
• **HSIAO, Fei-Yuan
10617 Taipei (TW)**

(74) Representative: **2K Patentanwälte Blasberg
Kewitz & Reichel
Partnerschaft mbB
Schumannstrasse 27
60325 Frankfurt am Main (DE)**

(54) **METHOD FOR CALCULATING HIGH RISK ROUTE OF ADMINISTRATION**

(57) A method for calculating a high risk route of administration is provided. Multiple arrangement routes composed of every two medicines among multiple medicines included in a medical record database (121) are listed (S205). A risk value of each arrangement route is calculated by querying the medical record database (121) based on a specified medication result (S210). A risk score of each arrangement route is calculated according to the risk value, and the arrangement routes are sorted based on the risk scores (S215). Starting from the arrangement route with the highest risk score, N arrangement routes are retrieved and a combination on N of the arrangement routes is performed to obtain multiple strung routes (S220). The number of medicines included in each strung route matches a specified medication number.

EP 4 020 496 A1

FIG. 2

**Description**

BACKGROUND

Technical Field

The disclosure relates to a method for searching a route of use among multiple medicines, and particularly relates to a method for calculating a high risk route of administration.

Description of Related Art

In recent years, due to the aging of the population and the prevalence of chronic diseases, it has become common to take two or more medicines at the same time or to combine medicines, Chinese herbal medicines or health foods, which both complicate administration. When the effect of a medicine is changed by other medicines, resulting in a decrease or increase in efficacy, it is called drug interaction. Mild drug interaction may only affect absorption of the medicines and affect the efficacy. However, in severe cases, drug interaction may be fatal. The chain effect of drug interaction may go through many medicines, and the patient may have seen more than one doctor, making it difficult to trace the cause. Therefore, if it is possible to find a high risk route of administration from the patient's medical record data, it will be easier for the doctor to pay attention to the administration history of the patient to avoid adverse chain effects. However, due to the wide variety of medicines, as the route of administration gets longer, the arrangement and combination may show a non-linear growth, resulting in a huge amount of data. Therefore, an efficient way is needed in order to find the high risk route.

SUMMARY

The disclosure provides a method for calculating a high risk route of administration, which saves time and avoids searching for a large number of routes of administration.

A method for calculating a high risk route of administration according to the disclosure includes: listing a plurality of arrangement routes composed of every two medicines among a plurality of medicines included in a medical record database; calculating a risk value of each of the arrangement routes by querying the medical record database based on a specified medication result; calculating a risk score of each of the arrangement routes according to the risk value, and sorting the arrangement routes based on the risk score; and retrieving N of the arrangement routes, starting from the arrangement route with a highest risk score, and performing a combination on N of the arrangement routes to obtain a plurality of strung routes. The number of medicines included in each of the strung routes matches a specified medication number.

In an embodiment of the disclosure, after retrieving N of the arrangement routes, starting from the arrangement route with the highest risk score, and performing a combination on N of the arrangement routes to obtain the plurality of strung routes, the method further includes: setting a preset number; determining whether the number of patients matching the obtained plurality of strung routes and having the specified medication result matches the preset number; and if the number of patients does not match the preset number, updating N to N+M, and re-executing retrieving N of the arrangement routes, starting from the arrangement route with the highest risk score, and performing a combination on N of the arrangement routes to obtain the plurality of strung routes until the number of patients matches the preset number.

In an embodiment of the disclosure, the risk value is an odds ratio, and the odds ratio of an $i^{th}$ arrangement route is calculated based on a following formula:

$$O = \frac{D_E/H_E}{D_N/H_N},$$

wherein O represents the odds ratio of the $i^{th}$ arrangement route, $D_E$ is a number of patients with medicine use records matching the $i^{th}$ arrangement route and causing the specified medication result, $H_E$ is a number of patients with medicine use records matching the $i^{th}$ arrangement route and not causing the specified medication result, $D_N$ is a number of patients with medicine use records not including the $i^{th}$ arrangement route and causing the specified medication result, and $H_N$ is a number of patients with medicine use records not including the $i^{th}$ arrangement route and not causing the specified medication result.

In an embodiment of the disclosure, calculating the risk score of each of the arrangement routes according to the risk value includes: setting a risk ranking of each of the arrangement routes based on the odds ratio of each of the arrangement routes; calculating a probability value of each of the arrangement routes, and setting a probability ranking of each of the arrangement routes based on the probability value; and adding the risk ranking to the probability ranking to obtain the risk score.

In an embodiment of the disclosure, the method for calculating the high risk route of administration further includes: querying for a number of patients matching the $i^{th}$ arrangement route in the medical record database based on a setting. The $i^{th}$ arrangement route sequentially includes a first medicine and a second medicine, and the setting is: querying for a number of patients who sequentially take the first medicine and the second medicine within a specified time range in medicine use records of a plurality of patients.

In an embodiment of the disclosure, the method for calculating the high risk route of administration further includes: retrieving a plurality of routes of administration recorded in the medical record database from the plurality of strung routes by querying the medical record database, and obtaining a data set of a plurality of diseases corresponding to a plurality of patients having the specified medication result, wherein ach of the plurality of patients corresponds to one of the plurality of diseases and has a route set, and the route set includes at least one of the routes of administration; calculating an inverse document frequency value of each of the routes of administration based on a following formula,

$$IDF(i) = \log\left(\frac{D}{t(i)}\right),$$

wherein IDF(i) represents the inverse document frequency value of an $i^{th}$ route of administration, D is a total number of the diseases, and t(i) is a number of the $i^{th}$ routes of administration included in the data set;

calculating an appearance frequency of each of the routes of administration in the route set corresponding to each of the patients based on a following formula,

$$TF_{i,j} = \frac{n_{i,j}}{A_j},$$

wherein $TF_{i,j}$ represents the appearance frequency of the $i^{th}$ route of administration in the route set corresponding to a $j^{th}$ patient, $n_{i,j}$ represents a number of the $i^{th}$ routes of administration that appear in the route set corresponding to the $j^{th}$ patient, and $A_j$ represents a number of routes of administration included in the route set corresponding to the $j^{th}$ patient;
calculating an estimated value of the $i^{th}$ route of administration corresponding to each of the patients based on the inverse document frequency value and the appearance frequency of each of the routes of administration; and selecting a unique route for each of the diseases based on the estimated value.

In an embodiment of the disclosure, selecting the unique route for each of the diseases includes: calculating an average value based on the estimated values of all the patients corresponding to each of the routes of administration; and taking the route of administration with a greatest average value as the unique route.

In an embodiment of the disclosure, selecting the unique route for each of the diseases includes: calculating an average value based on the estimated values of all the patients corresponding to each of the routes of administration; determining a threshold value based on an elbow method; and selecting all the routes of administration with average values greater than the threshold value as the unique route.

Based on the above, the disclosure provides a method for efficiently finding a high risk route, which saves time and avoids searching for a large number of routes of administration.

BRIEF DESCRIPTION OF THE DRAWINGS

The accompanying drawings are included to provide a further understanding of the disclosure, and are incorporated in and constitute a part of this specification. The drawings illustrate exemplary embodiments of the disclosure and, together with the description, serve to explain the principles of the disclosure.

FIG. 1 is a block diagram of an electronic device according to an embodiment of the disclosure.
FIG. 2 is a flowchart of a method for calculating a high risk route of administration according to an embodiment of the disclosure.

FIG. 3 is a flowchart of a method for finding unique routes of different diseases according to an embodiment of the disclosure.

FIG. 4 is a graph of average values of estimated values of routes of administration according to an embodiment of the disclosure.

## DETAILED DESCRIPTION OF DISCLOSED EMBODIMENTS

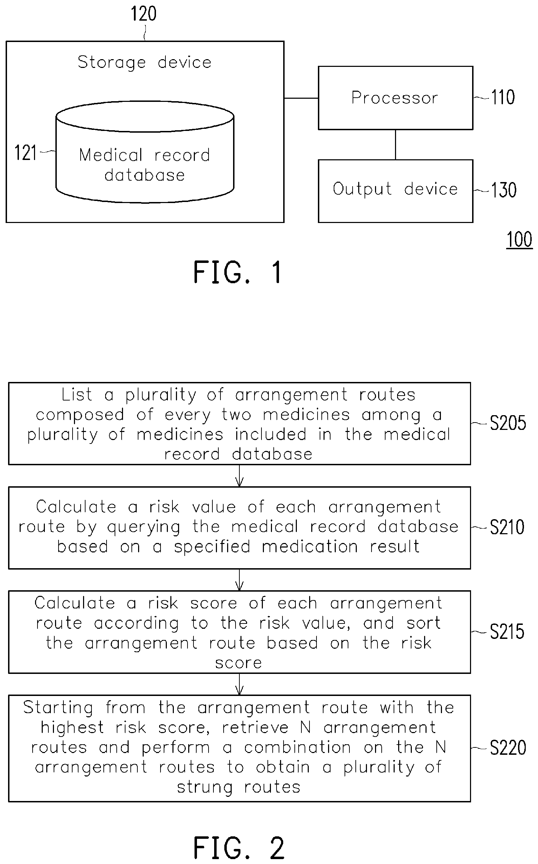

FIG. 1 is a block diagram of an electronic device according to an embodiment of the disclosure. Referring to FIG. 1, the electronic device 100 includes a processor 110, a storage device 120, and an output device 130. The processor 110 is coupled to the storage device 120 and the output device 130. The processor 110 is, for example, a central processing unit (CPU), a physics processing unit (PPU), a programmable microprocessor, an embedded control chip, a digital signal processor (DSP), an application specific integrated circuit (ASIC) or other similar devices.

The storage device 120 is, for example, any type of fixed or movable random access memory (RAM), read-only memory (ROM), flash memory, hard disk, other similar devices or a combination of these devices. A medical record database 121 records medicine use records of a plurality of patients. In other embodiments, the medical record database 121 may also be set on a cloud server, and the medical record database 121 may be downloaded from the cloud server to the electronic device 100 in advance, or the electronic device 100 may connect to the cloud server in real time to query the medical record database 121. In addition, the storage device 120 also stores a plurality of code snippets. After being installed, the code snippets are executed by the processor 110 to implement a method for calculating a high risk route of administration, as described below.

The method for calculating a high risk route of administration according to the disclosure is for finding an administration route, which is likely to cause a specified medication result, among multiple medicines. In the following embodiment, assuming that the specified medication number is 3, the representation of the route of administration is A→B→C, which means that the patient takes medicines in the order that the patient takes medicine A first, takes medicine B within a specified time range (for example, 30 days) after taking medicine A, and takes medicine C within a specified time range (for example, 30 days) after taking medicine B. The patient may also take other medicines after taking medicine A and before taking medicine B, and take other medicines after taking medicine B and before taking medicine C.

FIG. 2 is a flowchart of the method for calculating a high risk route of administration according to an embodiment of the disclosure. Referring to FIG. 2, in step S205, a plurality of arrangement routes composed of every two medicines among a plurality of medicines included in the medical record database 121 are listed. The processor 110 executes the relevant code snippets to find out each used medicine recorded in the medical record database 121, and forms a plurality of arrangement routes in a group of two.

For example, assuming that the medicine use records of a plurality of patients recorded in the medical record database 121 include a total of K medicines, K×(K-1) arrangement routes are obtained. There is a difference in the order of use between the two medicines included in each arrangement route. Take medicine A10BA, medicine C10AA, and medicine D01AC as an example, 6 arrangement routes are obtained, that is, A10BA→C10AA, A10BA→D01AC, C10AA→A10BA, C10AA→D01AC, D01AC→A10BA, and D01AC→C10AA.

Next, in step S210, a risk value of each arrangement route is calculated by querying the medical record database 121 based on a specified medication result. Here, the risk value may be calculated by using an odds ratio or logistic regression. In this embodiment, the odds ratio is used as the risk value. That is, the odds ratio O of the $i^{th}$ arrangement route is calculated based on the following formula:

$$O = \frac{D_E/H_E}{D_N/H_N}.$$

$D_E$ is the number of patients with medicine use records matching the $i^{th}$ arrangement route and causing the specified medication result, $H_E$ is the number of patients with medicine use records matching the $i^{th}$ arrangement route and not causing the specified medication result, $D_N$ is the number of patients with medicine use records not including the $i^{th}$ arrangement route and causing the specified medication result, and $H_N$ is the number of patients with medicine use records not including the $i^{th}$ arrangement route and not causing the specified medication result.

In terms of the arrangement route A10BA→C10AA (take medicine A10BA first and then take medicine C10AA), the medical record database 121 is queried for the medicine use record of each patient to find the patients with the arrangement route A10BA→C10AA and the patients without the arrangement route A10BA→C10AA. Among the patients with the arrangement route A10BA→C10AA, the number $D_E$ of patients who were hospitalized and the number $H_E$ of patients who were not hospitalized are found out. In addition, among the patients without the arrangement route A10BA→C10AA, the number $D_N$ of patients who were hospitalized and the number $H_N$ of patients who were not hospitalized are found out. Furthermore, the risk value of the arrangement route A10BA→C10AA (the odds ratio $O_{A10BA→C10AA}$) is obtained based on the above formula. Accordingly, the risk value corresponding to each arrangement route is calculated.

Here, the medical record database 121 is queried for the number of patients matching the ith arrangement route (sequentially including the first medicine and the second medicine) based on the following setting. The setting is: query for the number of patients who sequentially took the first medicine and the second medicine within a specified time range (for example, 30 days) in the medicine use records of a plurality of patients.

Next, in step S215, a risk score of each arrangement route is calculated according to the risk value, and the arrangement route is sorted based on the risk score. Furthermore, the risk ranking of each arrangement route is set based on the odds ratio of each arrangement route, and a probability value (p value) of each arrangement route is calculated, and the probability ranking of each arrangement route is set based on the p value. Then, the risk ranking is added to the probability ranking to obtain the risk score. For example, Table 1 shows the risk ranking, probability ranking, and risk score (risk ranking+probability ranking) corresponding to the arrangement route according to an embodiment.

Table 1

| Arrangement route | Risk ranking | Probability ranking | Risk score |
|---|---|---|---|
| A10BA→C10AA | 1 | 17 | 18 |
| ... | | | |
| D01AC→H03BA | 20 | 14 | 34 |
| ... | ... | ... | ... |
| N07AA→S01LA | 15 | 8 | 23 |

After the risk score is obtained, in step S220, starting from the arrangement route with the highest risk score, N arrangement routes are retrieved and a combination on N of the arrangement routes is performed to obtain a plurality of strung routes. Here, the number of medicines included in each strung route matches a specified medication number. For example, assuming that the specified medication number is 3, it means that the strung route is composed of two arrangement routes. For example, the arrangement route A→B and the arrangement route B→C may be combined into the strung route A→B→C.

In this embodiment, a higher risk score means that the order of taking medicines represented by the arrangement route is more likely to cause the specified medication result (for example, hospitalization). The strung route obtained from arrangement routes with higher risk scores usually has a higher risk of hospitalization. Accordingly, finding a high risk route of administration starting from the arrangement routes with high risk scores saves the time of searching all routes.

Specifically, an initial value of N and a stop condition of the search are set first. Here, the stop condition of the search is that the number of patients matching the obtained strung route and having the specified medication result matches a preset number. For example, the preset number is 50% of the total number of hospitalized patients. Then, the processor 110 queries the medical record database 121 for the medicine use record of each patient to find the total number of patients who were hospitalized, and determines whether the number of patients matching the obtained strung route and having the specified medication result matches the preset number. If the number of patients matching the obtained strung route and having the specified medication result does not match the preset number, the setting is set to N=N+M, and the step of retrieving N arrangement routes starting from the arrangement route with the highest risk score and performing a combination on N of the arrangement routes to obtain the strung route matching the specified medication number is re-executed, until the number of patients matching the obtained strung route and having the specified medication result matches the preset number. If N+M is greater than the obtained arrangement routes, all the arrangement routes are retrieved and the combination on all the arrangement routes is performed to obtain a plurality of strung routes matching the specified medication number.

Table 2 shows the search results of searching the arrangement routes. In two arrangement routes that are strung, the second medicine in the first arrangement route needs to be the same as the first medicine in the second arrangement route so as to string the two arrangement routes. Take the arrangement routes R05FA→N02BE, N02BE→A02BA, and N02BE→M01AB in Table 2 as an example, the strung routes obtained therefrom are R05FA→N02BE→A02BA and R05FA→N02BE→M01AB.

Table 2

| N value | Arrangement route | Obtained strung route | Search result |
|---|---|---|---|
| 100 | R05FA→N02BE<br><br>N02BE→A02BA<br><br>N02BE→M01AB<br><br>... | R05FA→N02BE→A02BA<br><br>R05FA→N02BE→M01AB<br><br>... | 7% |
| 200 | R05FA→N02BE<br>N02BE→A02BA<br>N02BE→M01AB<br>...<br>N02BE→H02AB<br>A03FA→R05CB<br>R05CB→B05XA<br>R05CB→A06AD<br>... | R05FA→N02BE→A02BA<br>R05FA→N02BE→M01AB<br>...<br>R05FA→N02BE→H02AB<br>A03FA→R05CB→B05XA<br>A03FA→R05CB→A06AD<br>... | 14% |
| ... | ... | ... | ... |
| 1400 | ... | ... | 46% |
| 1500 | ... | ... | 53% |

Regarding Table 2, the preset number is 50% of the total number of hospitalized patients, the initial value of N is 100, and M=100. When N is 100, the search result, that is, the number of patients matching the obtained strung route and having the specified medication result is only 7%. Therefore, N is reset to 200. When N is 1500, the search result (53%) is greater than the preset number 50%. Therefore, the stop condition is met and the search is stopped.

After the final strung route is obtained, unique routes of administration (hereinafter referred to as unique routes) may be further found for different diseases. In the following embodiment, the unique routes of different diseases are found by using term frequency and inverse document frequency.

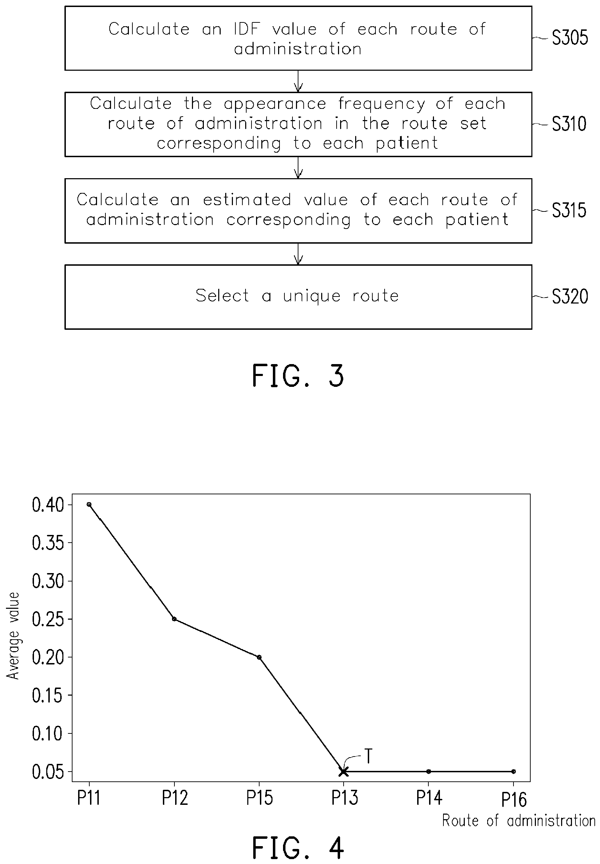

FIG. 3 is a flowchart of a method for finding unique routes of different diseases according to an embodiment of the disclosure. Referring to FIG. 3, in step S305, an IDF value of each route of administration is calculated. Specifically, the processor 110 retrieves a plurality of routes of administration recorded in the medical record database 121 from the strung route by querying the medical record database 121, and obtains data sets of a plurality of diseases corresponding to a plurality of patients having the specified medication result.

For example, assuming that the strung routes include A1 to A10, the processor 110 determines whether the strung routes A1 to A10 exist in the medical record database 121 by querying the medical record database 121 so as to find routes matching the strung routes A1 to A10 as the routes of administration. Here, it is assumed that the routes of administration P1 to P5 that match the strung routes A1 to A5 exist in the medical record database 121, and it is assumed that the specified medication result is hospitalization. Then, the patients who have the routes of administration P1 to P5 and have hospitalization records, and the diseases (causes of hospitalization) caused by these routes of administration are retrieved to obtain the data sets.

For example, Table 3 shows the data set according to an embodiment. As shown in Table 3, each patient corresponds to a disease (cause of hospitalization) and has a corresponding route set, and the route set includes at least one route of administration. In the data set shown in Table 3, patient numbers are used to distinguish different patients. The diseases listed here include pneumonia, peptic ulcer, and stroke, but not limited thereto. Take the patient number U01 as an example, the patient of the patient number U01 corresponds to pneumonia, and the route set includes routes of administration P1, P2, and P4.

Table 3

| Patient number | Disease (cause of hospitalization) | Route set |
|---|---|---|
| U01 | Pneumonia | {P1; P2; P4} |
| U02 | Pneumonia | {P3} |

(continued)

| Patient number | Disease (cause of hospitalization) | Route set |
|---|---|---|
| U03 | Peptic ulcer | {P1; P4} |
| U04 | Stroke | {P2; P4; P5} |
| U05 | Stroke | {P3; P4} |

After obtaining the data set, the processor 110 calculates the IDF value of each route of administration based on the following formula (1).

$$\text{Formula (1):} \quad IDF(i) = \log\left(\frac{D}{t(i)}\right).$$

IDF(i) represents the IDF value of the $i^{th}$ route of administration, D is the total number of diseases, and t(i) is the number of the $i^{th}$ routes of administration included in the data set. Take Table 3 as an example, the routes of administration include P1 to P5, and D=3. Referring to the route of administration P1, the number t(P1) of the routes of administration P1 in the data set is 2, so IDF(P1) = log(3/2) = 0.41. Accordingly, the IDF values of the routes of administration P2 to P5 are obtained respectively, as shown in Table 4. Here, a greater IDF value means that the corresponding route of administration appears in fewer causes of hospitalization (diseases) and is more unique.

Table 4

| Route of administration | P1 | P2 | P3 | P4 | P5 |
|---|---|---|---|---|---|
| IDF(i) | log(3/2) = 0.41 | log(3/2) = 0.41 | log(3/2) = 0.41 | log(3/3) = 0 | log(3/1) = 1.1 |

Next, in step S310, the processor 110 calculates the appearance frequency of each route of administration in the route set corresponding to each patient. Here, a term frequency calculation method is used to calculate the appearance frequency. That is, the appearance frequency of each route of administration in the route set corresponding to each patient is calculated based on the following formula (2).

$$\text{Formula (2):} \quad TF_{i,j} = \frac{n_{i,j}}{A_j}.$$

$TF_{i,j}$ represents the appearance frequency of the $i^{th}$ route of administration in the route set corresponding to the $j^{th}$ patient, $n_{i,j}$ represents the number of the $i^{th}$ routes of administration in the route set corresponding to the $j^{th}$ patient, and $A_j$ represents the number of routes of administration included in the route set corresponding to the $j^{th}$ patient.
Referring to Table 3, the route set {P1; P2; P4} of the patient number U01 includes 3 routes of administration. That is, $A_{U01}$ is 3. The number $n_{P1,U01}$ of the routes of administration P1 in the route set of the patient number U01 is 1. Accordingly, $TF_{P1,U01}$ is 1/3. The number $n_{P2,U01}$ of the routes of administration P2 in the route set of the patient number U01 is 1. Accordingly, $TF_{P2,U01}$ is 1/3. The number $n_{P3,U01}$ of the routes of administration P3 in the route set of the patient number U01 is 0. Accordingly, $TF_{P3,U01}$ is 0/3. The number $n_{P4,U01}$ of the routes of administration P4 in the route set of the patient number U01 is 1. Accordingly, $TF_{P4,U01}$ is 1/3. The number $n_{P5,U01}$ of the routes of administration P5 in the route set of the patient number U01 is 0. Accordingly, $TF_{P5,U01}$ is 0/3. Based on the above calculation, the appearance frequency $TF_{i,j}$ of each route set as shown in Table 5 is obtained.
Then, in step S315, the processor 110 calculates an estimated value of each route of administration corresponding to each patient. That is, based on the IDF value and appearance frequency of each route of administration, the estimated value of the $i^{th}$ route of administration corresponding to each patient is calculated. Here, the estimated value = $TF_{i,j} \times IDF(i)$. For IDF(i) in Table 4 and $TF_{i,j}$ in Table 5, the estimated values shown in Table 6 are obtained.

Table 5

| Appearance frequency $TF_{i,j}$ | | Route of administration | | | | |
|---|---|---|---|---|---|---|
| | | P1 | P2 | P3 | P4 | P5 |
| Patient number | U01 | 1/3 | 1/3 | 0 | 1/3 | 0 |
| | U02 | 0 | 0 | 1 | 0 | 0 |
| | U03 | 1/2 | 0 | 0 | 1/2 | 0 |
| | U04 | 0 | 1/3 | 0 | 1/3 | 1/3 |
| | U05 | 0 | 0 | 1/2 | 1/2 | 0 |

Table 6 ($TF_{i,j} \times IDF(i)$)

| Estimated value $TF_{i,j} \times IDF(i)$ | | Route of administration | | | | |
|---|---|---|---|---|---|---|
| | | P1 | P2 | P3 | P4 | P5 |
| Patient number | U01 | $1/3 \times 0.41$ | $1/3 \times 0.41$ | $0 \times 0.41$ | $1/3 \times 0$ | $0 \times 1.1$ |
| | U02 | $0 \times 0.41$ | $0 \times 0.41$ | $1 \times 0.41$ | $0 \times 0$ | $0 \times 1.1$ |
| | U03 | $1/2 \times 0.41$ | $0 \times 0.41$ | $0 \times 0.41$ | $1/2 \times 0$ | $0 \times 1.1$ |
| | U04 | $0 \times 0.41$ | $1/3 \times 0.41$ | $0 \times 0.41$ | $1/3 \times 0$ | $1/3 \times 1.1$ |
| | U05 | $0 \times 0.41$ | $0 \times 0.41$ | $1/2 \times 0.41$ | $1/2 \times 0$ | $0 \times 1.1$ |

In step S320, the processor 110 selects a unique route. That is, based on the estimated value, a unique route is selected for each disease. Here, the processor 110 calculates an average value based on the estimated values of all the patients corresponding to each route of administration, and uses the route of administration with the greatest average value as the unique route.

Referring to Table 3 and Table 6, the values for pneumonia are as shown in Table 7, and the values for stroke are as shown in Table 8. According to Table 7, the unique route selected for pneumonia is P3, which means that in the case of hospitalization due to pneumonia, the route of administration P3 has a high risk and has a low probability of resulting in other causes of hospitalization. According to Table 8, the unique route selected for stroke is P5, which means that in the case of hospitalization due to stroke, the route of administration P5 has a high risk and has a low probability of resulting in other causes of hospitalization. In addition, based on the example of Table 3, peptic ulcer only corresponds to the patient number U03. Therefore, referring to Table 6, the route of administration P1 corresponding to the maximum value among the estimated values corresponding to the patient number U03 is taken as the unique route.

Table 7

| Route of administration / Patient number | P1 | P2 | P3 | P4 | P5 |
|---|---|---|---|---|---|
| U01 | 0.136 | 0.136 | 0 | 0 | 0 |
| U02 | 0 | 0 | 0.41 | 0 | 0 |
| Average value | 0.068 | 0.068 | 0.205 | 0 | 0 |

Table 8

| Route of administration / Patient number | P1 | P2 | P3 | P4 | P5 |
|---|---|---|---|---|---|
| U04 | 0 | 0.136 | 0 | 0 | 0.367 |
| U05 | 0 | 0 | 0.205 | 0 | 0 |
| Average value | 0 | 0.068 | 0.1025 | 0 | 0.1835 |

In another method, the average value is calculated based on the estimated values of all the patients corresponding to each route of administration, and after a threshold value is selected by using an elbow method, all the routes of administration with an average value greater than the threshold value are selected as the unique routes. Another example is provided below. In the embodiment shown in Table 9, it is assumed that the cause of hospitalization is pneumonia, the patients include Ua, Ub, and Uc, and the routes of administration include P11 to P16.

Table 9

| Estimated value | | Route of administration | | | | | |
|---|---|---|---|---|---|---|---|
| | | P11 | P12 | P13 | P14 | P15 | P16 |
| Patient number | Ua | 0.35 | 0.3 | 0 | 0.05 | 0.2 | 0.1 |
| | Ub | 0.45 | 0.2 | 0.1 | 0.05 | 0.2 | 0 |
| | Uc | 0.4 | 0.25 | 0.05 | 0.05 | 0.2 | 0.05 |
| Average value | | 0.4 | 0.25 | 0.05 | 0.05 | 0.2 | 0.05 |

The average value is calculated based on the estimated values of the routes of administration P11 to P16 corresponding to all the patients shown in Table 9, and a graph is obtained based on these average values, as shown in FIG. 4. FIG. 4 is a graph of the average values of the estimated values of the routes of administration according to an embodiment of the disclosure. In FIG. 4, an elbow position (corresponding to the route of administration P13) in the graph is obtained as a threshold value T by using an elbow method, and the routes of administration with average values greater than the threshold value T are selected as the unique routes. That is, the routes of administration P11, P12, and P15 are determined as the unique routes.

In summary, the disclosure finds a high risk route of administration, which causes the specified medication result, by using the above-described calculation method, and can also find the unique routes that result in the specified medication result for different causes.

Claims

1. A method for calculating a high risk route of administration, **characterized in** comprising:

listing a plurality of arrangement routes composed of every two medicines among a plurality of medicines included in a medical record database (121) (S205);
calculating a risk value of each of the arrangement routes by querying the medical record database (121) based on a specified medication result (S210);
calculating a risk score of each of the arrangement routes according to the risk value, and sorting the arrangement routes based on the risk score (S215); and
retrieving N of the arrangement routes, starting from the arrangement route with a highest risk score, and performing a combination on N of the arrangement routes to obtain a plurality of strung routes, wherein a number of medicines included in each of the strung routes matches a specified medication number (S220).

2. The method for calculating the high risk route of administration according to claim 1, wherein after retrieving N of the arrangement routes, starting from the arrangement route with the highest risk score, and performing a combination on N of the arrangement routes to obtain the plurality of strung routes, the method further comprises:

> setting a preset number;
> determining whether a number of patients matching the obtained plurality of strung routes and having the specified medication result matches the preset number; and
> if the number of patients does not match the preset number, updating N to N+M, and re-executing retrieving N of the arrangement routes, starting from the arrangement route with the highest risk score, and performing a combination on N of the arrangement routes to obtain the plurality of strung routes until the number of patients matches the preset number.

3. The method for calculating the high risk route of administration according to claim 1, wherein the risk value is an odds ratio, and the odds ratio of an $i^{th}$ arrangement route is calculated based on a following formula:

$$O = \frac{D_E/H_E}{D_N/H_N},$$

> wherein O represents the odds ratio of the $i^{th}$ arrangement route,
> $D_E$ is a number of patients with medicine use records matching the $i^{th}$ arrangement route and causing the specified medication result,
> $H_E$ is a number of patients with medicine use records matching the $i^{th}$ arrangement route and not causing the specified medication result,
> $D_N$ is a number of patients with medicine use records not including the $i^{th}$ arrangement route and causing the specified medication result, and
> $H_N$ is a number of patients with medicine use records not including the $i^{th}$ arrangement route and not causing the specified medication result.

4. The method for calculating the high risk route of administration according to claim 3, wherein calculating the risk score of each of the arrangement routes according to the risk value comprises:

> setting a risk ranking of each of the arrangement routes based on the odds ratio of each of the arrangement routes;
> calculating a probability value of each of the arrangement routes, and setting a probability ranking of each of the arrangement routes based on the probability value; and
> adding the risk ranking to the probability ranking to obtain the risk score.

5. The method for calculating the high risk route of administration according to claim 1, further comprising:
querying for a number of patients matching the $i^{th}$ arrangement route in the medical record database (121) based on a setting, wherein the $i^{th}$ arrangement route sequentially comprises a first medicine and a second medicine, and the setting is: querying for a number of patients who sequentially take the first medicine and the second medicine within a specified time range in medicine use records of a plurality of patients.

6. The method for calculating the high risk route of administration according to claim 1, further comprising:

> retrieving a plurality of routes of administration recorded in the medical record database (121) from the plurality of strung routes by querying the medical record database (121), and obtaining a data set of a plurality of diseases corresponding to a plurality of patients having the specified medication result (S305), wherein each of the plurality of patients corresponds to one of the plurality of diseases and has a route set, and the route set comprises at least one of the routes of administration;
> calculating an inverse document frequency value of each of the routes of administration based on a following formula,

$$IDF(i) = \log\left(\frac{D}{t(i)}\right),$$

wherein IDF(i) represents the inverse document frequency value of an $i^{th}$ route of administration, D is a total number of the diseases, and t(i) is a number of the $i^{th}$ routes of administration included in the data set; calculating an appearance frequency of each of the routes of administration in the route set corresponding to each of the patients based on a following formula (S310),

$$TF_{i,j} = \frac{n_{i,j}}{A_j},$$

wherein $TF_{i,j}$ represents the appearance frequency of the $i^{th}$ route of administration in the route set corresponding to a $j^{th}$ patient, $n_{i,j}$ represents a number of the $i^{th}$ routes of administration that appear in the route set corresponding to the $j^{th}$ patient, and $A_j$ represents a number of routes of administration included in the route set corresponding to the $j^{th}$ patient; calculating an estimated value of the $i^{th}$ route of administration corresponding to each of the patients based on the inverse document frequency value and the appearance frequency of each of the routes of administration (S315); and selecting a unique route for each of the diseases based on the estimated value (S320).

7. The method for calculating the high risk route of administration according to claim 6, wherein selecting the unique route for each of the diseases comprises:

calculating an average value based on the estimated values of all the patients corresponding to each of the routes of administration; and taking the route of administration with a greatest average value as the unique route.

8. The method for calculating the high risk route of administration according to claim 6, wherein selecting the unique route for each of the diseases comprises:

calculating an average value based on the estimated values of all the patients corresponding to each of the routes of administration; determining a threshold value based on an elbow method; and selecting all the routes of administration with average values greater than the threshold value as the unique route.

120

Storage device

121 — Medical record database

Processor —110

Output device —130

100

# FIG. 1

List a plurality of arrangement routes composed of every two medicines among a plurality of medicines included in the medical record database — S205

Calculate a risk value of each arrangement route by querying the medical record database based on a specified medication result — S210

Calculate a risk score of each arrangement route according to the risk value, and sort the arrangement route based on the risk score — S215

Starting from the arrangement route with the highest risk score, retrieve N arrangement routes and perform a combination on the N arrangement routes to obtain a plurality of strung routes — S220

# FIG. 2

| Calculate an IDF value of each route of administration | ~S305 |
| Calculate the appearance frequency of each route of administration in the route set corresponding to each patient | ~S310 |
| Calculate an estimated value of each route of administration corresponding to each patient | ~S315 |
| Select a unique route | ~S320 |

FIG. 3

FIG. 4

Europäisches
Patentamt

European
Patent Office

Office européen
des brevets

# EUROPEAN SEARCH REPORT

Application Number

EP 21 19 6267

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | HUIDONG JIN ET AL: "Mining Unexpected Temporal Associations: Applications in Detecting Adverse Drug Reactions", IEEE TRANSACTIONS ON INFORMATION TECHNOLOGY IN BIOMEDICINE, IEEE SERVICE CENTER, LOS ALAMITOS, CA, US, vol. 12, no. 4, 1 July 2008 (2008-07-01), pages 488-500, XP011246413, ISSN: 1089-7771 * abstract; figures 1, 2 * * II. PROBLEM FORMULATION III. SEARCHING FOR UNEXPECTED TEMPORAL ASSOCIATION RULES VI. CONCLUSION AND DISCUSSION * ----- | 1-8 | INV. G16H50/70 |
| X | US 2020/312434 A1 (TURGEON JACQUES [US] ET AL) 1 October 2020 (2020-10-01) * paragraphs [0405], [0415]; claim 1; figure 1 * ----- | 1 | |
| X | US 2006/036619 A1 (FUERST OREN [US] ET AL) 16 February 2006 (2006-02-16) * claim 1; figure 1 * ----- | 1 | TECHNICAL FIELDS SEARCHED (IPC) G16H |

The present search report has been drawn up for all claims

| Place of search | Date of completion of the search | Examiner |
|---|---|---|
| Munich | 16 February 2022 | Beligny, Samuel |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons
 
& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 21 19 6267

This annex lists the patent family members relating to the patent documents cited in the above-mentioned European search report.
The members are as contained in the European Patent Office EDP file on
The European Patent Office is in no way liable for these particulars which are merely given for the purpose of information.

16-02-2022

| Patent document cited in search report | | Publication date | Patent family member(s) | | Publication date |
|---|---|---|---|---|---|
| US 2020312434 | A1 | 01-10-2020 | US 2020312434 A1 | | 01-10-2020 |
| | | | WO 2021226489 A1 | | 11-11-2021 |
| US 2006036619 | A1 | 16-02-2006 | NONE | | |

EPO FORM P0459

For more details about this annex : see Official Journal of the European Patent Office, No. 12/82